# EUROPEAN PATENT APPLICATION

(11) **EP 4 059 474 A1**
(43) Date of publication of application: **21.09.2022**
(21) Application number: 22162116.2
(22) Date of filing: 15.03.2022
(51) Int. Cl.: A61F 2/04, A61F 2/86

(54) **STENTS AND METHODS OF MAKING AND USING THE SAME**

(30) Priority: 15.03.2021 US 202163161211 P
(71) Applicant: The Corporation Of Mercer University, Macon, Georgia 31207 (US)
(72) Inventor: THOMAS, Joanna L., Macon, 31201 (US); PATEL, Sagar, G., Perry, 31069 (US)
(74) Representative: Heubeck, Christian

(57) **Abstract**

Stents are disclosed. The stents provide novel stent designs that can, in some cases, span bifurcations in a duct, such as the extrahepatic bile duct (EHBD) (e.g., where the right hepatic duct meets the left hepatic duct), without impeding bile flow from either duct branch. Methods of making and using stents are also disclosed.

## Description

### FIELD OF THE INVENTION

The present invention is directed to stents such as gastrointestinal stents and biliary stents. The present invention is further directed to methods of making and using stents.

### BACKGROUND

The etiology of cholestasis is variable, but often it is caused by strictures in the extrahepatic bile ducts (EHBD). Nonpharmacological treatments for biliary strictures are self-expanding metal stents (SEMs) or plastic stents that are inserted during cholangiopancreatography to re-establish bile flow. Plastic stents, unlike SEMS, do not interfere with subsequent MRIs; however, if incorrectly placed, plastic stents block bile flow at bifurcations of the EHBD.

Efforts continue to further develop stents.

### SUMMARY

The present invention addresses some of the difficulties and problems in the art by the discovery of new stents. The stents described herein provide a novel stent design that can span bifurcations in the extrahepatic bile duct (EHBD) (e.g., where the right hepatic duct meets the left hepatic duct) without impeding bile flow from either duct branch. Accordingly, the present invention is directed to stents including gastrointestinal stents and biliary stents.

In one exemplary embodiment, the stent of the present invention comprises: (I) a first end tubular member comprising (i) a first end tubular member outer surface, (ii) a first end tubular member inner surface, (iii) a first end tubular member wall extending between the first end tubular member outer surface and the first end tubular member inner surface, and (iv) a first end tubular member lumen extending through the first end tubular member along the first end tubular member inner surface; (II) a second end tubular member comprising (i) a second end tubular member outer surface, (ii) a second end tubular member inner surface, (iii) a second end tubular member wall extending between the second end tubular member outer surface and the second end tubular member inner surface, and (iv) a second end tubular member lumen extending through the second end tubular member along the second end tubular member inner surface; and (III) a fluid-permeable section extending between and connecting said first end tubular member to said second end tubular member, said fluid-permeable section (a) comprising one or more struts with each strut extending between and connecting said first end tubular member to said second end tubular member, (b) allowing fluid flow from each of (i) said first end tubular member and (ii) said second end tubular member to pass into and through said fluid-permeable section to a location outside an outer boundary of said fluid-permeable section, and (c) having a compliance that is more than (i) said first end tubular member, and (ii) said second end tubular member.

In another exemplary embodiment, the stent of the present invention comprises: (I) a first end tubular member comprising (i) a first end tubular member outer surface, (ii) a first end tubular member inner surface, (iii) a first end tubular member wall extending between the first end tubular member outer surface and the first end tubular member inner surface, and (iv) a first end tubular member lumen extending through the first end tubular member along the first end tubular member inner surface; (II) a second end tubular member comprising (i) a second end tubular member outer surface, (ii) a second end tubular member inner surface, (iii) a second end tubular member wall extending between the second end tubular member outer surface and the second end tubular member inner surface, and (iv) a second end tubular member lumen extending through the second end tubular member along the second end tubular member inner surface; (III) a fluid-permeable section extending between and connecting said first end tubular member to said second end tubular member, said fluid-permeable section (a) comprising one or more struts with each strut extending between and connecting said first end tubular member to said second end tubular member, (b) allowing fluid flow from each of (i) said first end tubular member and (ii) said second end tubular member to pass into and through said fluid-permeable section to a location outside an outer boundary of said fluid-permeable section, and (c) having a compliance that is more than (i) said first end tubular member, and (ii) said second end tubular member; (IV) a third tubular member comprising (i) a third tubular member outer surface, (ii) a third tubular member inner surface, (iii) a third tubular member wall extending between the third tubular member outer surface and the third tubular member inner surface, and (iv) a third tubular member lumen extending through the third tubular member along the third tubular member inner surface; and (V) a second fluid-permeable section extending between and connecting said second tubular member to said third tubular member, said second fluid-permeable section (a) comprising one or more struts with each strut extending between and connecting said second tubular member to said third tubular member, (b) allowing fluid flow from each of (i) said second tubular member and (ii) said third tubular member to pass into and through said second fluid-permeable section to a location outside an outer boundary of said second fluid-permeable section, and (c) having a compliance that is more than (i) said second tubular member, and (ii) said third tubular member.

In yet another exemplary embodiment, the stent of the present invention comprises: (I) a first end tubular member comprising (i) a first end tubular member outer surface, (ii) a first end tubular member inner surface, (iii) a first end tubular member wall extending between the first end tubular member outer surface and the first end tubular member inner surface, and (iv) a first end tubular member lumen extending through the first end tubular member along the first end tubular member inner surface; (II) a second end tubular member comprising (i) a second end tubular member outer surface, (ii) a second end tubular member inner surface, (iii) a second end tubular member wall extending between the second end tubular member outer surface and the second end tubular member inner surface, and (iv) a second end tubular member lumen extending through the second end tubular member along the second end tubular member inner surface; (III) a fluid-permeable section extending between and connecting said first end tubular member to said second end tubular member, said fluid-permeable section (a) comprising one or more struts with each strut extending between and connecting said first end tubular member to said second end tubular member, (b) allowing fluid flow from each of (i) said first end tubular member and (ii) said second end tubular member to pass into and through said fluid-permeable section to a location outside an outer boundary of said fluid-permeable section, and (c) having a compliance that is more than (i) said first end tubular member, and (ii) said second end tubular member; (IV) a third tubular member comprising (i) a third tubular member outer surface, (ii) a third tubular member inner surface, (iii) a third tubular member wall extending between the third tubular member outer surface and the third tubular member inner surface, and (iv) a third tubular member lumen extending through the third tubular member along the third tubular member inner surface; (V) a second fluid-permeable section extending between and connecting said second tubular member to said third tubular member, said second fluid-permeable section (a) comprising one or more struts with each strut extending between and connecting said second tubular member to said third tubular member, (b) allowing fluid flow from each of (i) said second tubular member and (ii) said third tubular member to pass into and through said second fluid-permeable section to a location outside an outer boundary of said second fluid-permeable section, and (c) having a compliance that is more than (i) said second tubular member, and (ii) said third tubular member; (VI) a fourth tubular member comprising (i) a fourth tubular member outer surface, (ii) a fourth tubular member inner surface, (iii) a fourth tubular member wall extending between the fourth tubular member outer surface and the fourth tubular member inner surface, and (iv) a fourth tubular member lumen extending through the fourth tubular member along the fourth tubular member inner surface; and (VII) a third fluid-permeable section extending between and connecting said third tubular member to said fourth tubular member, said third fluid-permeable section (a) comprising one or more struts with each strut extending between and connecting said third tubular member to said fourth tubular member, (b) allowing fluid flow from each of (i) said third tubular member and (ii) said fourth tubular member to pass into and through said third fluid-permeable section to a location outside an outer boundary of said third fluid-permeable section, and (c) having a compliance that is more than (i) said third tubular member, and (ii) said fourth tubular member.

The present invention further relates to methods of making stents. In one exemplary embodiment, the method of making a stent comprises: 3D printing the herein described stent.

The present invention even further relates to methods of using stents. In one exemplary embodiment, the method of using a stent comprises positioning the herein described stent within a duct. In some desired embodiments, the method of using a stent comprises positioning the herein described stent within adjacent duct branches at a bifurcation in a duct, for example, in the extrahepatic bile duct (EHBD) (e.g., where the right hepatic duct meets the left hepatic duct) without impeding bile flow from either duct branch. The disclosed method of using a stent may be used, for example, by gastroenterologists in palliative, short-, or long-term treatments for cholestasis.

The present invention further relates to a stent CAD model, which can be used to rapidly tailor a specific stent, such as a gastrointestinal stent or a biliary stent, that precisely matches a patient's anatomy. The stent CAD model is compatible with standard plastic stent deployment apparatuses.

In addition, the herein described stent is readily modifiable. All stent dimensions can be adjusted in the CAD model to personalize a stent for unique patient anatomy. Like other plastic stents, the herein described stent is compatible with all imaging modalities.

These and other features and advantages of the present invention will become apparent after a review of the following detailed description of the disclosed embodiments and the appended claims.

### BRIEF DESCRIPTION OF THE FIGURES

The present invention is further described with reference to the appended figures, wherein:
FIG **1** is a perspective frontal view of an exemplary stent of the present invention;

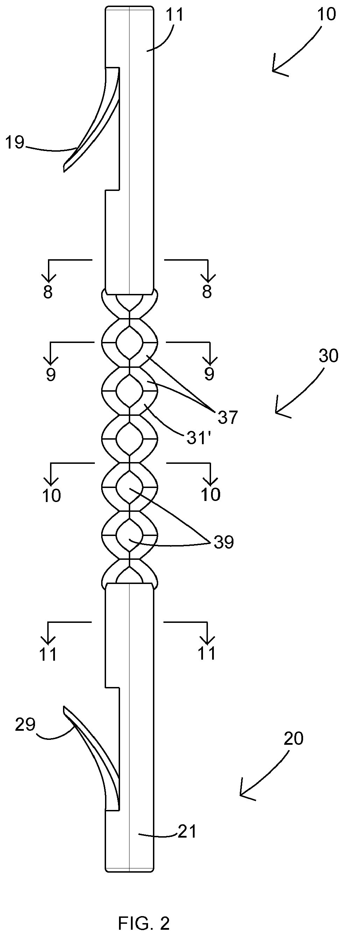

FIG. **2** is a right-hand view of the exemplary stent shown in FIG. **1**;

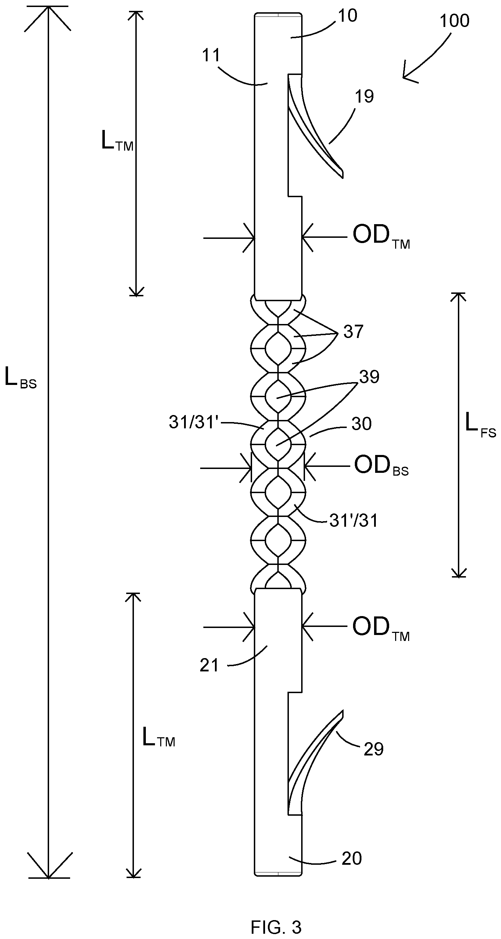

FIG. **3** is a left-hand view of the exemplary stent shown in FIG. **1**;
FIG. **4** is front view of the exemplary stent shown in FIG. **1**;
FIG. **5** is a rear view of the exemplary stent shown in FIG. **1**;

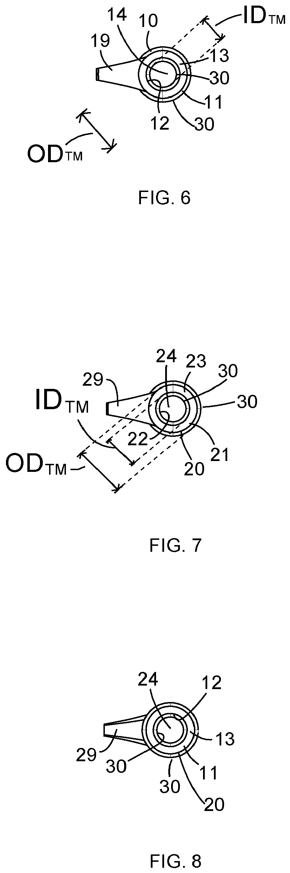

FIG. **6** is a top view of the exemplary stent shown in FIG. **1**;
FIG. **7** is a bottom view of the exemplary stent shown in FIG. **1**;
FIG. **8** is a cross-sectional view of the exemplary stent shown in FIG. **1** as viewed along line **8-8** shown in FIG. **2**;
FIG. **9** is a cross-sectional view of the exemplary stent shown in FIG. **1** as viewed along line **9-9** shown in FIG. **2**;
FIG. **10** is a cross-sectional view of the exemplary stent shown in FIG. **1** as viewed along line **10-10** shown in FIG. **2**;
FIG. **11** is a cross-sectional view of the exemplary stent shown in FIG. **1** as viewed along line **11-11** shown in FIG. **2**;
FIG. **12** is a perspective view of another exemplary stent of the present invention;
FIG. **13** is a cross-sectional view of the exemplary stent shown in FIG. **12** as viewed along line **13-13** shown in FIG. **12**;
FIG. **14** is a cross-sectional view of the exemplary stent shown in FIG. **12** as viewed along line **14-14** shown in FIG. **12**;

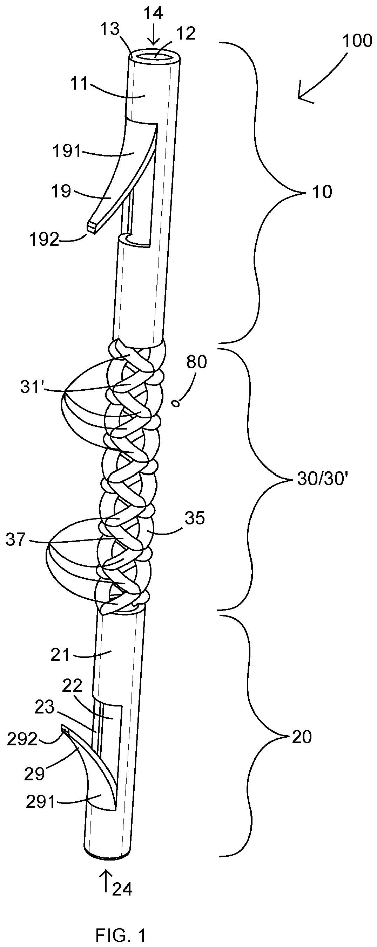

FIG. 1**5** is a side view of an exemplary 3D printing assembly for forming exemplary stents of the present invention such as the exemplary stent shown in FIG. **1**;

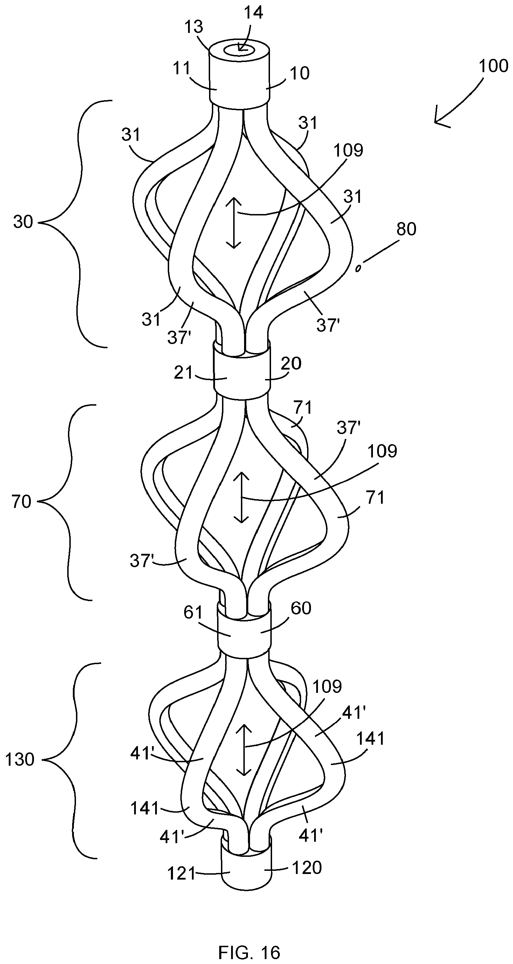

FIG. **16** is a perspective view of another exemplary stent of the present invention;

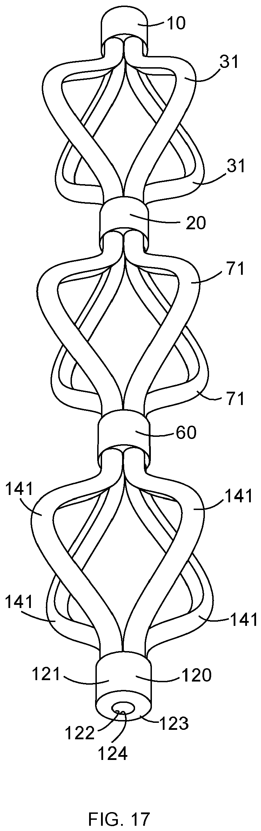

FIG. **17** is another perspective view of the exemplary stent shown in FIG. **16**;

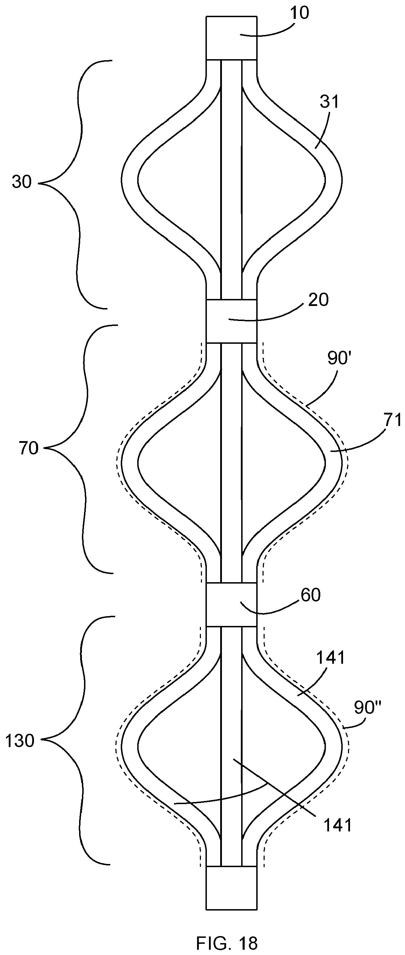

FIG. **18** represents (i) a front view, (ii) a rear view, (iii) a right-hand view, and (iv) a left-hand view of the exemplary stent shown in FIG. **16**;

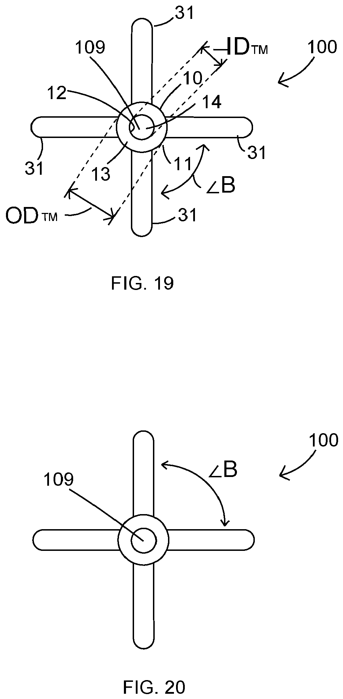

FIG. **19** is a top view of the exemplary stent shown in FIG. **16**;
FIG. **20** is a bottom view of the exemplary stent shown in FIG. **16**;

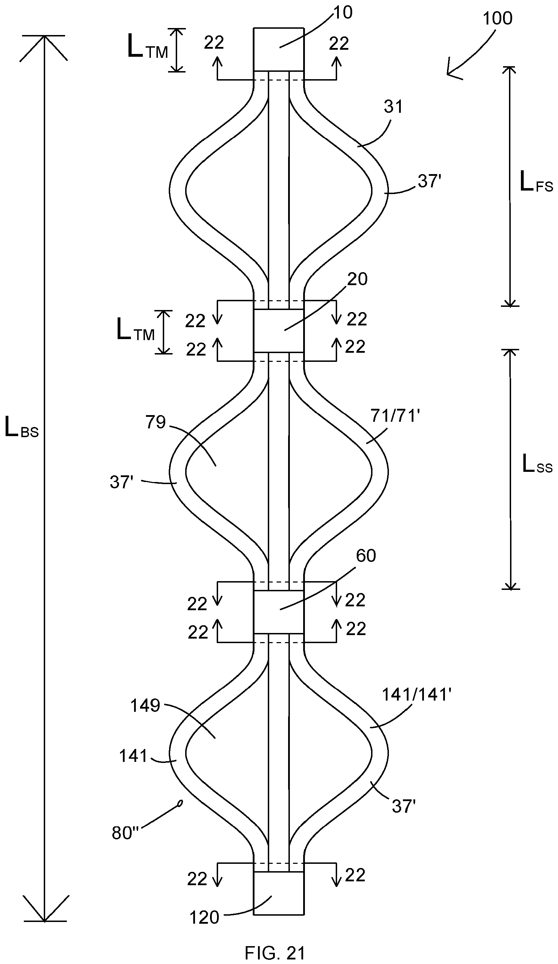

FIG. **21** is another front view of the exemplary stent shown in FIG. **16**; and

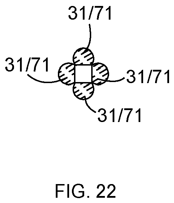

FIG. **22** is a cross-sectional view along each of lines **22-22** shown in FIG. **21****.**

### DETAILED DESCRIPTION

To promote an understanding of the principles of the present invention, descriptions of specific embodiments of the invention follow and specific language is used to describe the specific embodiments. It will nevertheless be understood that no limitation of the scope of the invention is intended by the use of specific language. Alterations, further modifications, and such further applications of the principles of the present invention discussed are contemplated as would normally occur to one ordinarily skilled in the art to which the invention pertains.

The present invention is directed to stents such as gastrointestinal stents and biliary stents. The present invention is further directed to methods of making stents such as gastrointestinal stents and biliary stents. The present invention is even further directed to methods of using stents such as gastrointestinal stents and biliary stents.

The stents of the present invention are further described in the following embodiments.

### Other Embodiments:

### Stents

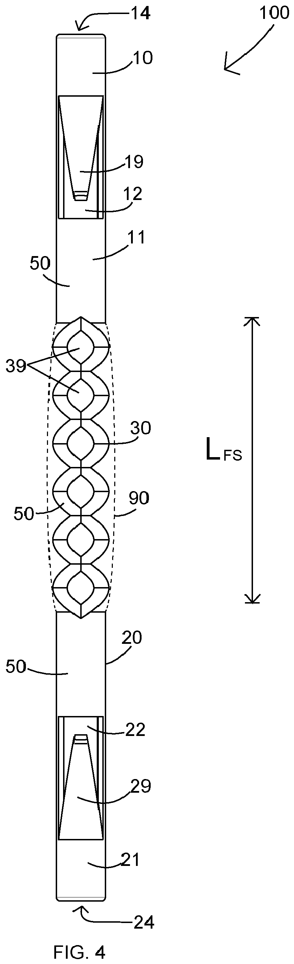

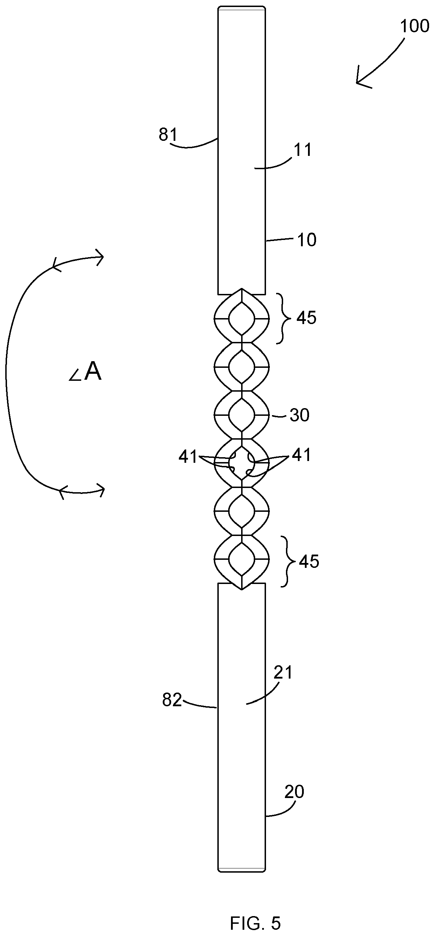

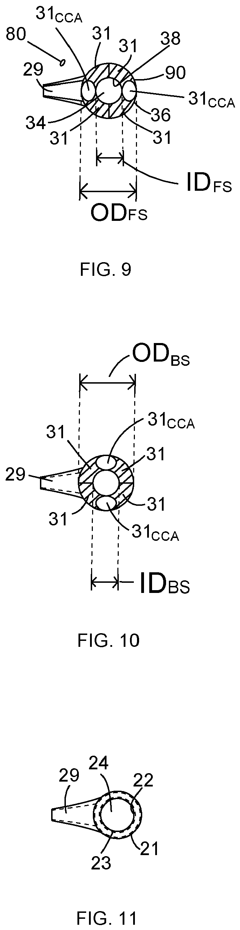

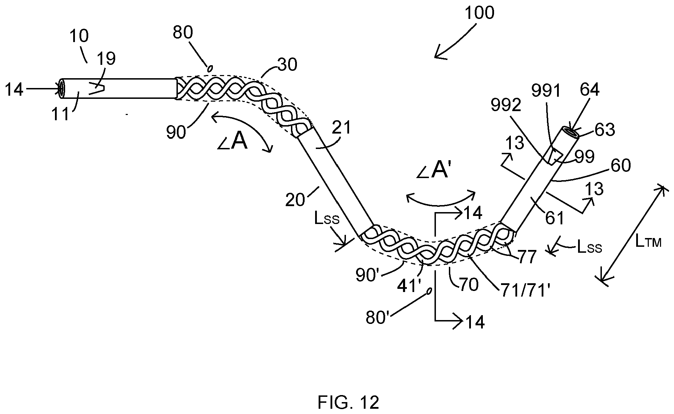

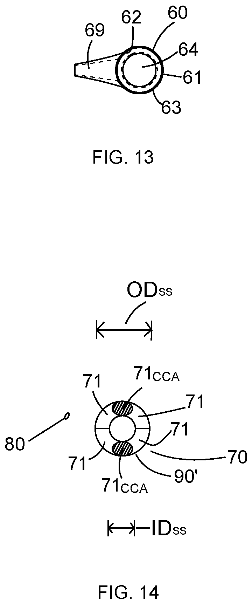

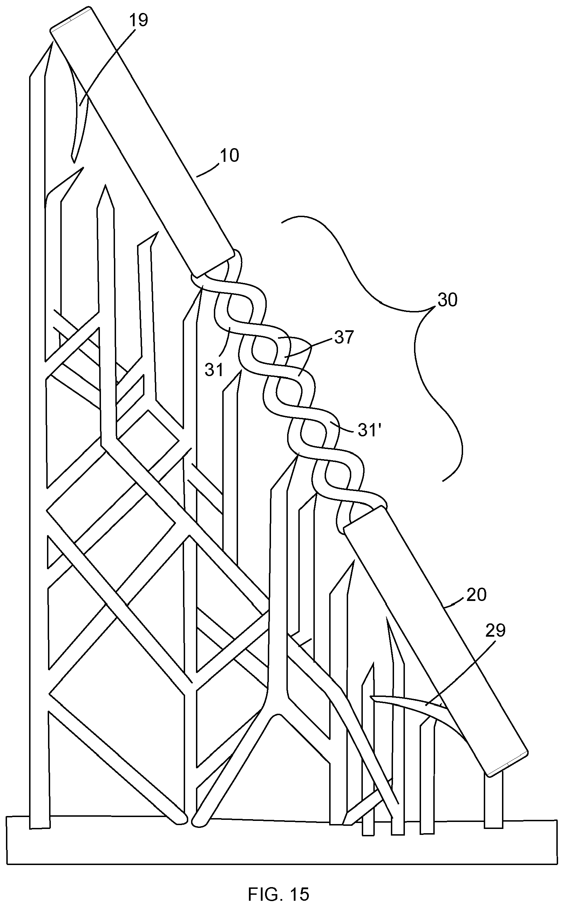

1. A stent **100** comprising: (I) a first end tubular member **10** comprising (i) a first end tubular member outer surface **11**, (ii) a first end tubular member inner surface **12**, (iii) a first end tubular member wall **13** extending between the first end tubular member outer surface **11** and the first end tubular member inner surface **12**, and (iv) a first end tubular member lumen **14** extending through the first end tubular member **10** along the first end tubular member inner surface **12**; (II) a second tubular member **20** comprising (i) a second tubular member outer surface **21**, (ii) a second tubular member inner surface **22**, (iii) a second tubular member wall **23** extending between the second tubular member outer surface **21** and the second tubular member inner surface **22**, and (iv) a second tubular member lumen **24** extending through the second tubular member **20** along the second tubular member inner surface **22**; and (III) a fluid-permeable section **30** extending between and connecting said first end tubular member **10** to said second tubular member **20**, said fluid-permeable section **30** (a) comprising one or more struts **31** with each strut **31** extending between and connecting said first end tubular member **10** to said second tubular member **20**, (b) allowing fluid flow from each of (i) said first end tubular member **10** and (ii) said second tubular member **20** to pass into and through said fluid-permeable section **30** to a location **80** outside an outer boundary **90** of said fluid-permeable section **30**, and (c) having a compliance that is more than (i) said first end tubular member **10**, and (ii) said second tubular member **20.** See, for example, FIGS. **1**, **12** and **16****.** As used herein, the term "compliance" is used to represent a rate at which an outer diameter of the fluid-permeable section **30**, the first end tubular member **10**, or the second tubular member **20** expands radially in response to a pressure change within the fluid-permeable section **30** or the first end tubular member **10** or the second tubular member **20.** Outer boundary **90** is shown in dashed lines in FIG. **4** and is represented by a cylindrical volume (i) extending between first end tubular member **10** and said second tubular member **20**, and (ii) having a volume diameter equal to or slightly larger than an outer diameter of first end tubular member **10** and/or second tubular member **20.** In some preferred embodiments, the stent **100** comprises a gastrointestinal stent **100** or a biliary stent **100.**
2. The stent **100** of embodiment 1, wherein each of said first end tubular member **10** and said second tubular member **20** independently has (i) a tubular member length **L_{TM}** of less than about 5.0 centimeters (cm), (ii) a tubular member outer diameter **OD_{TM}** of less than about 1.0 cm, and (iii) a tubular member inner diameter **ID_{TM}** of less than about 0.8 cm.
3. The stent **100** of embodiment 1 or 2, wherein each of said first end tubular member **10** and said second tubular member **20** independently has (i) a tubular member length **L_{TM}** of from about 0.5 cm to about 3.0 cm (or a tubular member length **L_{TM}** of from about 1.0 cm to about 3.0 cm), (ii) a tubular member outer diameter **OD_{TM}** of from about 0.3 cm to about 0.8 cm, and (iii) a tubular member inner diameter **ID_{TM}** of from about 0.1 cm to about 0.3 cm.
4. The stent **100** of any one of embodiments 1 to 3, wherein the one or more struts **31** have a combined strut cross-sectional area **31_{CCA}** that is (i) less than a first end tubular member cross-sectional area **10_{CA}**, and (ii) less than a second tubular member cross-sectional area **20_{CA}.** Note that combined strut cross-sectional area **31_{CCA}** equals the sum of the individual strut cross-sectional areas **31_{CCA}** of the one or more struts **31.** Further note that (1) each individual strut cross-sectional area **31_{CCA}** of the one or more struts **31** is represented by a cross-section of material **50** (e.g., a metal, or a 3D printable polymeric material **50**) used to form each strut **31** at a given location along the fluid-permeable section **30**, and (2) each (i) first end tubular member cross-sectional area **10_{CA}** and (ii) second tubular member cross-sectional area **20_{CA}** is independently represented by a cross-section of material **50** (e.g., a metal, or a 3D printable polymeric material **50**) used to form first end tubular member **10** or second tubular member **20** at a given location along first end tubular member **10** or second tubular member **20.**
5. The stent **100** of any one of embodiments 1 to 4, wherein the one or more struts **31** have a combined strut cross-sectional area **31_{CCA}** that (i) at least 50% less than the first end tubular member cross-sectional area **10_{CA}**, and (ii) at least 50% less than the second tubular member cross-sectional area **20_{CA}.**
6. The stent **100** of any one of embodiments 1 to 5, wherein each of the one or more struts **31** comprises a strand **31'** of interconnected pieces **37.**
7. The stent **100** of any one of embodiments 1 to 6, wherein each of the one or more struts **31** comprises a strand **31'** of from about four to about 24 interconnected pieces **37.**
8. The stent **100** of any one of embodiments 1 to 7, wherein each of the one or more struts **31** comprises a strand 31' of about 12 interconnected pieces 37.
9. The stent **100** of any one of embodiments 1 to 8, wherein each of the one or more struts **31** has a zig-zag configuration.
10. The stent **100** of any one of embodiments 1 to 9, wherein said fluid-permeable section **30** comprising one or more section units **45** with each section unit **45** comprising eight interconnected pieces **37.**
11. The stent **100** of any one of embodiments 1 to 10, wherein the one or more struts **31** form a tubular fluid-permeable section **30'**, said tubular fluid-permeable section **30'** comprising (i) a tubular fluid-permeable section grid **35** extending between a tubular fluid-permeable section outer surface **36** and the tubular fluid-permeable section inner surface **38**, and (iv) a tubular fluid-permeable section lumen **34** extending through the tubular fluid-permeable section **30'** along the tubular fluid-permeable section inner surface **38.** See, for example, FIG. **9****.**
12. The stent **100** of any one of embodiments 1 to 5, wherein each of the one or more struts **31** comprises a strand **31'** comprising a single strand piece **37'.**
13. The stent **100** of embodiment 12, wherein each of the single strand pieces **37'** are positioned a substantially equal distance (or an equal distance) from one another around a dissecting line **109** extending through the fluid-permeable section **30.** See, for example, FIGS. **16** and **19****.**
14. The stent **100** of any one of embodiments 1 to 13, wherein said fluid-permeable section **30** comprising two or more struts **31** (e.g., two or more strands **31'** of interconnected pieces **37** or two single strand pieces **37'**).
15. The stent **100** of any one of embodiments 1 to 14, wherein said fluid-permeable section **30** comprising from two to eight struts **31** (e.g., from two to eight strands **31'** of interconnected pieces **37** or from two to eight single strand pieces **37'**).
16. The stent **100** of any one of embodiments 1 to 15, wherein said fluid-permeable section **30** comprising four struts **31** (e.g., four strands **31'** of interconnected pieces **37** or four single strand pieces **37'**).
17. The stent **100** of embodiment 16, wherein each of the single strand pieces **37'** are separated from one another around dissecting line **109** by an angle **B**, wherein **B** ranges from about 75° to about 105°. See, for example, FIG. **19****.**
18. The stent **100** of embodiment 16 or 17, wherein each of the single strand pieces **37'** are separated from one another around dissecting line **109** by an angle **B**, wherein **B** is about 90°. See, for example, FIG. **19****.**
19. The stent **100** of any one of embodiments 1 to 18, wherein said fluid-permeable section **30** has (i) a fluid-permeable section length **L_{FS}** of less than about 5.0 cm, and (ii) a fluid-permeable section outer diameter **OD_{FS}** of less than about 3.0 cm (or less than about 2.5 cm, or less than about 2.0 cm, or less than about 1.5 cm, or less than about 1.0 cm). See again, for example, FIG. **9****.**
20. The stent **100** of any one of embodiments 1 to 11 and 14 to 19, wherein said fluid-permeable section **30** has (i) a fluid-permeable section length **L_{FS}** of from about 0.5 cm to about 3.5 cm, and (ii) a fluid-permeable section outer diameter **OD_{FS}** of from about 0.3 cm to about 0.8 cm.
21. The stent **100** of any one of embodiments 1 to 11 and 14 to 20, wherein said tubular fluid-permeable section **30** has an inner diameter **ID_{FS}** of less than about 0.8 cm.
22. The stent **100** of any one of embodiments 1 to 11 and 14 to 21, wherein said tubular fluid-permeable section **30** has an inner diameter **ID_{FS}** of from about 0.1 cm to about 0.3 cm.
23. The stent **100** of any one of embodiments 1 to 5 and 12 to 19, wherein said fluid-permeable section **30** has (i) a fluid-permeable section length **L_{FS}** of from about 0.5 cm to about 3.5 cm, and (ii) a fluid-permeable section outer diameter **OD_{FS}** of from about 0.5 cm to about 3.0 cm.
24. The stent **100** of any one of embodiments 1 to 5, 12 to 19, and 23, wherein said tubular fluid-permeable section **30** has an inner diameter **ID_{FS}** that varies from less than about 1.0 cm to greater than about 2.0 cm along said fluid-permeable section length **L_{FS}**.
25. The stent **100** of any one of embodiments 1 to 5, 12 to 19, and 23, wherein said tubular fluid-permeable section **30** has an inner diameter **ID_{FS}** that peaks in size at approximately a midpoint along said fluid-permeable section length **L_{FS}.**
26. The stent **100** of any one of embodiments 1 to 25, wherein said fluid-permeable section **30** comprises at least three fluid-permeable section openings **39** that provide fluid flow from inside said tubular fluid-permeable section **30** to location **80** outside of said tubular fluid-permeable section **30.**
27. The stent **100** of any one of embodiments 1 to 11, 14 to 22 and 26, wherein said fluid-permeable section **30** comprises from about 3 fluid-permeable section openings **39** to about 48 fluid-permeable section openings **39** that provide fluid flow from said tubular fluid-permeable section lumen **34** to location **80** outside of said tubular fluid-permeable section **30.**
28. The stent **100** of any one of embodiments 1 to 5, 12 to 19, 23 to 26, wherein said fluid-permeable section **30** comprises four fluid-permeable section openings **39** that provide fluid flow from inside said tubular fluid-permeable section **30** to location **80** outside of said tubular fluid-permeable section **30.**
29. The stent **100** of any one of embodiments 26 to 28, wherein each fluid-permeable section opening **39** has four opening sides **41.**
30. The stent **100** of any one of embodiments 1 to 29, wherein each of said first end tubular member outer surface **11** and said first end tubular member inner surface **12** is substantially continuous. As used herein, the phrase "substantially continuous" is used to describe a surface that does not have any openings or apertures therein (i.e., is fluid-impermeable) unless specifically mentioned (e.g., such as the herein-described optional flange members **19/29**).
31. The stent **100** of any one of embodiments 1 to 30, wherein each of said second tubular member outer surface **21** and said second tubular member inner surface **22** is substantially continuous.
32. The stent **100** of any one of embodiments 1 to 31, wherein said first end tubular member **10** further comprises a first end tubular member flange **19** extending outward from said first end tubular member outer surface **11.**
33. The stent **100** of embodiment 32, wherein said first end tubular member flange **19** comprises a first end tubular member flange connected end **191** and a first end tubular member flange engaging end **192**, the first end tubular member flange engaging end **192** being closer to the fluid-permeable section **30** than said first end tubular member flange connected end **191.**
34. The stent **100** of any one of embodiments 1 to 33, wherein said second tubular member **20** further comprises a second tubular member flange **29** extending outward from said second tubular member outer surface **21.**
35. The stent **100** of embodiment 34, wherein said second tubular member flange **29** comprises a second tubular member flange connected end **291** and a second tubular member flange engaging end **292**, the second tubular member flange engaging end **292** being closer to the fluid-permeable section **30** that said second end tubular member flange connected end **291.**
36. The stent **100** of any one of embodiments 1 to 35, wherein said second tubular member **20** represents an end tubular member opposite said first tubular member **10.**
37. The stent **100** of any one of embodiments 1 to 35, further comprising: (a) a third tubular member **60** comprising (i) a third tubular member outer surface **61**, (ii) a third tubular member inner surface **62**, (iii) a third tubular member wall **63** extending between the third tubular member outer surface **61** and the third tubular member inner surface **62**, and (iv) a third tubular member lumen **64** extending through the third tubular member **60** along the third tubular member inner surface **62**; and (III) a second fluid-permeable section **70** extending between and connecting said second tubular member **20** to said third tubular member **60**, said second fluid-permeable section **70** (a) comprising one or more struts **71** with each strut **71** extending between and connecting said second tubular member **20** to said third tubular member **60**, (b) allowing fluid flow from each of (i) said second tubular member **20** and (ii) said third tubular member **60** to pass into and through said second fluid-permeable section **70** to a location **80'** outside an outer boundary **90'** of said second fluid-permeable section **70**, and (c) having a compliance that is more than (i) said second tubular member **20**, and (ii) said third tubular member **60.** See, for example, FIGS. **12** and **18****.** In FIG. **12**, outer boundary **90'** is shown in dashed lines, and represents a cylindrical volume (i) extending between second tubular member **20** and third tubular member **60**, and (ii) having a volume diameter equal to or slightly larger than an outer diameter of second tubular member **20** and/or third tubular member **60.** In FIG. **18**, outer boundary **90'** is shown in dashed lines, and represents a cylindrical volume (i) extending between second tubular member **20** and third tubular member **60**, and (ii) having a volume diameter that varies from (a) being about equal to or less than an outer diameter of second tubular member **20** and/or third tubular member **60**, to (b) a peak volume diameter that exceeds the outer diameter of the second tubular member **20** and the third tubular member **60** at a point along a second fluid-permeable section length Lss between the second tubular member **20** and the third tubular member **60.**
38. The stent **100** of embodiment 37, wherein said third tubular member **60** has (i) a tubular member length **L_{TM}** of less than about 5.0 cm, (ii) a tubular member outer diameter **OD_{TM}** of less than about 1.0 cm, and (iii) a tubular member inner diameter **ID_{TM}** of less than about 0.8 cm.
39. The stent **100** of embodiment 37 or 38, wherein said third tubular member 60 has (i) a tubular member length **L_{TM}** of from about 0.5 cm to about 3.0 cm, (ii) a tubular member outer diameter **OD_{TM}** of from about 0.3 cm to about 0.8 cm, and (iii) a tubular member inner diameter **ID_{TM}** of from about 0.1 cm to about 0.3 cm.
40. The stent **100** of any one of embodiments 37 to 39, wherein the one or more strut **71** have a combined strut cross-sectional area **71_{CCA}** that is (i) less than a first end tubular member cross-sectional area **10_{CA}**, and (ii) less than a second tubular member cross-sectional area **20_{CA}.**
41. The stent **100** of any one of embodiments 37 to 40, wherein the one or more struts **71** have a combined strut cross-sectional area **71_{CCA}** that (i) at least 50% less than the first end tubular member cross-sectional area **10_{CA}**, and (ii) at least 50% less than the second tubular member cross-sectional area **20_{CA}.**
42. The stent **100** of any one of embodiments 37 to 41, wherein each of the one or more struts **71** comprises a strand **71'** of interconnected pieces 77.
43. The stent **100** of any one of embodiments 37 to 42, wherein each of the one or more struts **71** comprises a strand **71'** of from about four to about 24 interconnected piece 77.
44. The stent **100** of any one of embodiments 37 to 43, wherein each of the one or more struts **71** comprises a strand **71'** of about 12 interconnected pieces **77.**
45. The stent **100** of any one of embodiments 37 to 44, wherein each of the one or more struts **71** has a zig-zag configuration.
46. The stent **100** of any one of embodiments 37 to 45, wherein said second fluid-permeable section **70** comprising one or more section units **45** with each section unit **45** comprising eight interconnected pieces 77.
47. The stent **100** of any one of embodiments 37 to 46, wherein the one or more struts **71** form a second tubular fluid-permeable section **70'**, said second tubular fluid-permeable section **70'** comprising (i) a second tubular fluid-permeable section grid **75** extending between a second tubular fluid-permeable section outer surface **76** and the second tubular fluid-permeable section inner surface **78**, and (iv) a second tubular fluid-permeable section lumen **74** extending through the second tubular fluid-permeable section **70'** along the second tubular fluid-permeable section inner surface **38.** See, for example, FIG. **12****.**
48. The stent **100** of any one of embodiments 37 to 41, wherein each of the one or more struts **71** comprises a strand **71'** comprising a single strand piece **37'.**
49. The stent **100** of embodiment 48, wherein each of the single strand pieces **37'** are positioned a substantially equal distance (or an equal distance) from one another around a dissecting line **109** extending through the fluid-permeable section **70.** See, for example, FIG. **16****.**
50. The stent **100** of any one of embodiments 37 to 49, wherein said second fluid-permeable section **70** comprising two or more struts **71** (e.g., two or more strands **71'** of interconnected pieces **77** or two single strand pieces **37'**).
51. The stent **100** of any one of embodiments 37 to 50, wherein said second fluid-permeable section **70** comprising from two to eight struts **71** (e.g., from two to eight strands **71'** of interconnected pieces **77** or from two to eight single strand pieces **37'**).
52. The stent **100** of any one of embodiments 37 to 51, wherein said second fluid-permeable section **70** comprising four struts **71** (e.g., four strands **71'** of interconnected pieces **77** or four single strand pieces **37'**).
53. The stent **100** of embodiment 52, wherein each of the single strand pieces **37'** are separated from one another around dissecting line **109** by an angle **B**, wherein **B** ranges from about 75° to about 105°. See, for example, FIG. **19****.**
54. The stent **100** of embodiment 52 or 53, wherein each of the single strand pieces **37'** are separated from one another around dissecting line **109** by an angle **B**, wherein **B** is about 90°. See, for example, FIG. **19****.**
55. The stent **100** of embodiment 52 or 53, wherein each of the one or more struts **71** within said second fluid-permeable section **70** is aligned with each of the one or more struts **31** within said fluid-permeable section **30.** See, for example, FIG. **16****.** See, for example, FIG. **16****.**
56. The stent **100** of any one of embodiments 37 to 55, wherein said second fluid-permeable section **70** has (i) a second fluid-permeable section length **L_{SS}** of less than about 5.0 cm, and (ii) a second fluid-permeable section outer diameter **OD_{SS}** of less than about 3.0 cm (or less than about 2.5 cm, or less than about 2.0 cm, or less than about 1.5 cm, or less than about 1.0 cm). See again, for example, FIGS. **12** and **16****.**
57. The stent **100** of any one of embodiments 37 to 47, 50 to 52 and 56, wherein said second fluid-permeable section **70** has (i) a second fluid-permeable section length **L_{SS}** of from about 0.5 cm to about 3.5 cm, and (ii) a second fluid-permeable section outer diameter **OD_{SS}** of from about 0.3 cm to about 0.8 cm.
58. The stent **100** of any one of embodiments 37 to 47, 50 to 52 and 56 to 57, wherein said second tubular fluid-permeable section **70** has an inner diameter **ID_{SS}** of less than about 0.8 cm. See again, for example, FIGS. **12** and **14****.**
59. The stent **100** of any one of embodiments 37 to 47, 50 to 52 and 56 to 58, wherein said second tubular fluid-permeable section **70** has an inner diameter **ID_{FS}** of from about 0.1 cm to about 0.3 cm.
60. The stent **100** of any one of embodiments 37 to 41, and 48 to 56, wherein said second tubular fluid-permeable section **70** has (i) a second fluid-permeable section length **L_{SS}** of from about 0.5 cm to about 3.5 cm, and (ii) a second fluid-permeable section outer diameter **OD_{FS}** of from about 0.5 cm to about 3.0 cm.
61. The stent **100** of any one of embodiments 37 to 41, 48 to 56, and 60, wherein said second tubular fluid-permeable section **70** has an inner diameter **ID_{FS}** that varies from less than about 1.0 cm to greater than about 2.0 cm along said second fluid-permeable section length **L_{SS}.**
62. The stent **100** of any one of embodiments 37 to 41, 48 to 56, and 60 to 61, wherein said second tubular fluid-permeable section **70** has an inner diameter **ID_{FS}** that peaks in size at approximately a midpoint along said second fluid-permeable section length **L_{SS}.**
63. The stent **100** of any one of embodiments 37 to 62, wherein said second fluid-permeable section **70** comprises at least three second fluid-permeable section openings **79** that provide fluid flow from inside said second tubular fluid-permeable section **70** to location **80'** outside of said second tubular fluid-permeable section **70.**
64. The stent **100** of any one of embodiments 37 to 47, 50 to 52, and 63, wherein said second fluid-permeable section **70** comprises from about 3 second fluid-permeable section openings **79** to about 48 second fluid-permeable section openings **79** that provide fluid flow from inside said second tubular fluid-permeable section **70** to location **80'** outside of said second tubular fluid-permeable section **70.**
65. The stent **100** of any one of embodiments 37 to 41, 48 to 52, and 60 to 64, wherein said second fluid-permeable section **70** comprises four fluid-permeable section openings **79** that provide fluid flow from inside said second tubular fluid-permeable section **70** to location **80** outside of said second tubular fluid-permeable section **70.**
66. The stent **100** of any one of embodiments 63 to 65, wherein each second fluid-permeable section opening **79** has four opening sides **41'.**
67. The stent **100** of any one of embodiments 37 to 66, wherein each of said third tubular member outer surface **61** and said third tubular member inner surface **62** is substantially continuous.
68. The stent **100** of any one of embodiments 37 to 67, wherein said third tubular member **60** further comprises a third tubular member flange **99** extending outward from said third tubular member outer surface **61.**
69. The stent **100** of embodiment 68, wherein said third tubular member flange **99** comprises a third tubular member flange connected end **991** and a third tubular member flange engaging end **992**, the third tubular member flange engaging end **992** being closer to the second fluid-permeable section **70** than said third tubular member flange connected end **991.**
70. The stent **100** of any one of embodiments 37 to 69, wherein said third tubular member **60** represents an end tubular member opposite said first tubular member **10.**
71. The stent **100** of any one of embodiments 37 to 69, further comprising: (a) a fourth tubular member **120** comprising (i) a fourth tubular member outer surface **121**, (ii) a fourth tubular member inner surface **122**, (iii) a fourth tubular member wall **123** extending between the fourth tubular member outer surface **121** and the fourth tubular member inner surface **122**, and (iv) a fourth tubular member lumen **124** extending through the fourth tubular member **120** along the fourth tubular member inner surface **122**; and (III) a third fluid-permeable section **130** extending between and connecting said third tubular member **60** to said fourth tubular member **120**, said third fluid-permeable section **130** (a) comprising one or more struts **141** with each strut **141** extending between and connecting said third tubular member **60** to said fourth tubular member **120**, (b) allowing fluid flow from each of (i) said third tubular member **60** and (ii) said fourth tubular member **120** to pass into and through said third fluid-permeable section **70** to a location **80'** outside an outer boundary **90"** of said third fluid-permeable section **130**, and (c) having a compliance that is more than (i) said third tubular member **60**, and (ii) said fourth tubular member **120.** In FIG. **18**, outer boundary **90"** is shown in dashed lines, and represents a cylindrical volume (i) extending between third tubular member **60** and fourth tubular member **120**, and (ii) having a volume diameter that varies from (a) being about equal to or less than an outer diameter of the third tubular member **60** and/or the fourth tubular member **120**, to (b) a peak volume diameter that exceeds the outer diameter of the third tubular member **60** and the fourth tubular member **120** at a point along a third fluid-permeable section length **L_{TS}** between the third tubular member **60** and the fourth tubular member **120.**
72. The stent **100** of embodiment 71, wherein said fourth tubular member **120** has (i) a tubular member length **L_{TM}** of less than about 5.0 cm, (ii) a tubular member outer diameter **OD_{TM}** of less than about 1.0 cm, and (iii) a tubular member inner diameter **ID_{TM}** of less than about 0.8 cm.
73. The stent **100** of embodiment 71 or 72, wherein said fourth tubular member **120** has (i) a tubular member length **L_{TM}** of from about 1.0 cm to about 3.0 cm, (ii) a tubular member outer diameter **OD_{TM}** of from about 0.3 cm to about 0.8 cm, and (iii) a tubular member inner diameter **ID_{TM}** of from about 0.1 cm to about 0.3 cm.
74. The stent **100** of any one of embodiments 71 to 73, wherein the one or more strut **141** have a combined strut cross-sectional area **141_{CCA}** that is (i) less than a third end tubular member cross-sectional area **60_{CA},** and (ii) less than a fourth tubular member cross-sectional area **120_{CA}.**
75. The stent **100** of any one of embodiments 71 to 74, wherein the one or more struts **141** have a combined strut cross-sectional area **141_{CCA}** that is (i) at least 50% less than the third end tubular member cross-sectional area **60_{CA},** and (ii) at least 50% less than the fourth tubular member cross-sectional area **120_{CA}.**
76. The stent **100** of any one of embodiments 71 to 75, wherein each of the one or more struts **141** comprises a strand **141'** comprising a single strand piece **37'.**
77. The stent **100** of embodiment 76, wherein each of the single strand pieces **37'** are positioned a substantially equal distance (or an equal distance) from one another around a dissecting line **109** extending through the third fluid-permeable section **130.** See, for example, FIG. **16****.**
78. The stent **100** of any one of embodiments 71 to 77, wherein said third fluid-permeable section **130** comprising two or more struts **141** (e.g., two or more single strand pieces **37'**).
79. The stent **100** of any one of embodiments 71 to 78, wherein said third fluid-permeable section **130** comprising from two to eight struts **141** (e.g., from two to eight single strand pieces **37'**).
80. The stent **100** of any one of embodiments 71 to 79, wherein said third fluid-permeable section **130** comprising four struts **141** (e.g., four strands **141'** of four single strand pieces **37'**).
81. The stent **100** of embodiment 80, wherein each of the single strand pieces **37'** are separated from one another around dissecting line **109** by an angle **B**, wherein **B** ranges from about 75° to about 105°. See, for example, FIG. **19****.**
82. The stent **100** of embodiment 80 or 81, wherein each of the single strand pieces **37'** are separated from one another around dissecting line **109** by an angle **B**, wherein **B** is about 90°. See, for example, FIG. **19****.**
83. The stent **100** of embodiment 80 or 53, wherein each of the one or more struts **141** within said third fluid-permeable section **130** is aligned with (i) each of the one or more struts **31** within said fluid-permeable section **30**, and (ii) each of the one or more struts **71** within said second fluid-permeable section **70.** See, for example, FIG. **16****.**
84. The stent **100** of any one of embodiments 71 to 83, wherein said third fluid-permeable section **130** has (i) a third fluid-permeable section length **L_{SS}** of less than about 5.0 cm, and (ii) a third fluid-permeable section outer diameter **OD_{SS}** of less than about 3.0 cm (or less than about 2.5 cm, or less than about 2.0 cm, or less than about 1.5 cm, or less than about 1.0 cm). See again, for example, FIG. **16****.**
85. The stent **100** of any one of embodiments 71 to 84, wherein said third tubular fluid-permeable section **130** has (i) a third fluid-permeable section length Lss of from about 0.5 cm to about 3.5 cm, and (ii) a third fluid-permeable section outer diameter **OD_{FS}** of from about 0.5 cm to about 3.0 cm.
86. The stent **100** of any one of embodiments 71 to 85, wherein said third tubular fluid-permeable section **130** has an inner diameter **ID_{FS}** that varies from less than about 1.0 cm to greater than about 2.0 cm along said third fluid-permeable section length Lss.
87. The stent **100** of any one of embodiments 71 to 86, wherein said third tubular fluid-permeable section **130** has an inner diameter **ID_{FS}** that peaks in size at approximately a midpoint along said third fluid-permeable section length **L_{SS}.**
88. The stent **100** of any one of embodiments 71 to 87, wherein said third fluid-permeable section **130** comprises at least three third fluid-permeable section openings **149** that provide fluid flow from inside said third tubular fluid-permeable section **130** to location **80"** outside of said third tubular fluid-permeable section **130.**
89. The stent **100** of any one of embodiments 71 to 88, wherein said third fluid-permeable section **130** comprises four fluid-permeable section openings **149** that provide fluid flow from inside said third tubular fluid-permeable section **130** to location **80"** outside of said third tubular fluid-permeable section **130.**
90. The stent **100** of embodiment 88 or 89, wherein each third fluid-permeable section opening **149** has four opening sides **41'.**
91. The stent **100** of any one of embodiments 71 to 90, wherein each of said fourth tubular member outer surface **121** and said fourth tubular member inner surface **122** is substantially continuous.
92. The stent **100** of any one of embodiments 71 to 91, wherein said fourth tubular member **120** further comprises a fourth tubular member flange (not shown) extending outward from said fourth tubular member outer surface **121.**
93. The stent **100** of embodiment 92, wherein said fourth tubular member flange comprises a fourth tubular member flange connected end (not shown) and a fourth tubular member flange engaging end (not shown), the fourth tubular member flange engaging end being closer to the third fluid-permeable section **130** than said fourth tubular member flange connected end.
94. The stent **100** of any one of embodiments 71 to 93, wherein said fourth tubular member **120** represents an end tubular member opposite said first tubular member **10.** See again, for example, FIG. **16****.**
95. The stent **100** of any one of embodiments 1 to 94, wherein said stent **100** has (i) an overall length **L_{BS}** of less than about 10.0 cm, (ii) an overall outer diameter **OD_{BS}** of less than about 3.0 cm, and (iii) an overall inner diameter **ID_{BS}** of less than about 3.0 cm. In some embodiments, the overall outer diameter **OD_{BS}** is substantially equal to the fluid-permeable section outer diameter **OD_{FS}.** In some embodiments, the overall outer diameter **OD_{BS}** is substantially equal to the tubular member outer diameter **OD_{TM}.** In some embodiments, the overall inner diameter **ID_{BS}** is substantially equal to the fluid-permeable section inner diameter **ID_{FS}.** In some embodiments, the overall inner diameter **ID_{BS}** is substantially equal to the tubular member inner diameter **ID_{TM}**. In some embodiments, the fluid-permeable section inner diameter **ID_{FS}** is greater than the tubular member inner diameter **ID_{TM}.**
96. The stent **100** of any one of embodiments 1 to 95, wherein said stent **100** has (i) an overall length **L_{BS}** of from about 1.0 cm to about 3.0 cm, (ii) an overall outer diameter **OD_{BS}** of from about 0.3 cm to about 2.8 cm, and (iii) an overall inner diameter **ID_{BS}** of from about 0.1 cm to about 2.8 cm.
97. The stent **100** of any one of embodiments 1 to 96, wherein said stent **100** comprises a polymeric material **50.**
98. The stent **100** of any one of embodiments 1 to 97 wherein said stent **100** comprises a polyolefin material **50.**
99. The stent **100** of any one of embodiments 1 to 98, wherein said stent **100** comprises a polypropylene material **50.**
100. The stent **100** of any one of embodiments 1 to 96, wherein said stent **100** comprises a metal material **50.**
101. The stent **100** of any one of embodiments 1 to 97 and 100, wherein said stent **100** comprises a shape memory material having elastic properties such that when the fluid-permeable section **30** (and/or the first fluid-permeable section **70** and/or the third fluid-permeable section **130**) is compressed from an initial configuration via an outside pressure applied onto the fluid-permeable section **30** (and/or the first fluid-permeable section **70** and/or the third fluid-permeable section **130**), the fluid-permeable section **30** (and/or the first fluid-permeable section **70** and/or the third fluid-permeable section **130**) returns to its initial configuration once the outside pressure is removed (i.e., not applied).
102. The stent **100** of any one of embodiments 1 to 101, wherein said stent **100** comprises a 3D printable material **50.** In some embodiments, stent **100** comprises a 3D printable material **50**, namely, Formlabs Durable Resin from Formlabs, Inc. (Somerville, MA, USA).
103. The stent **100** of any one of embodiments 1 to 102, wherein said stent **100** is bendable along the fluid-permeable section **30** such that said first end tubular member **10** and said second tubular member **20** form an angle **A** therebetween, the angle **A** ranging from 0° to 180°. As shown in FIG. **5**, angle **A** is 180°. When angle **A** moves closer to 0°, point **81** along first end tubular member **10** moves closer to point **82** along second tubular member **20.** See again, FIG. **5****.**
104. The stent **100** of any one of embodiments 37 to 103, wherein said stent **100** provides unobstructed fluid flow (i) through said second tubular member lumen **24** into said second fluid-permeable section **70** and out of said second fluid-permeable section **70**, and (ii) through said third tubular member lumen **74** into said second fluid-permeable section **70** and out of said second fluid-permeable section **70.**
105. The stent **100** of any one of embodiments 37 to 104, wherein said stent **100** is bendable along the second fluid-permeable section **70** such that said second tubular member **20** and said third tubular member **60** form an angle **A'** therebetween, the angle **A'** ranging from 0° to 180°.
106. The stent **100** of any one of embodiments 37 to 105, wherein said stent **100** provides unobstructed fluid flow (i) through said second tubular member lumen **24** into said second fluid-permeable section **70** and out of said second fluid-permeable section **70**, and (ii) through said third tubular member lumen **74** into said second fluid-permeable section **70** and out of said second fluid-permeable section **70.**
107. The stent **100** of any one of embodiments 1 to 106, wherein said stent **100** comprises a gastrointestinal stent **100.**
108. The stent **100** of any one of embodiments 1 to 107, wherein said stent **100** comprises a biliary stent **100.**

### Methods of Making Stents

109. A method of making the stent **100** of any one of embodiments 1 to 108, said method comprising: forming the first end tubular member **10**, forming the second tubular member **20**, and forming the fluid-permeable section **30.**

110. The method of embodiment 109, further comprising: forming the third tubular member **60**, and forming the second fluid-permeable section **70.**

111. The method of embodiment 109 or 110, further comprising: forming the fourth tubular member **120**, and forming the third fluid-permeable section **130.**

112. The method of any one of embodiments 109 to 111, wherein said forming steps occur simultaneously.

113. The method of any one of embodiments 109 to 112, wherein said forming steps comprise: using a stent CAD model (not shown) to design a specific stent **100** that precisely matches a specific patient's anatomy (not shown).

114. The method of any one of embodiments 109 to 113, wherein said forming steps comprise a 3D printing step.

115. The method of any one of embodiments 109 to 114, wherein said forming steps comprise 3D printing a polymeric material **50** or a metal material **50.**

116. The method of any one of embodiments 109 to 115, wherein said forming steps comprise 3D printing a polypropylene material **50.**

117. The method of any one of embodiments 109 to 116, further comprising: washing the stent **100** in an isopropanol wash.

118. The method of any one of embodiments 109 to 117, further comprising: air drying the stent **100.**

119. The method of any one of embodiments 109 to 118, further comprising: UV curing the stent **100.**

120. The method of any one of embodiments 109 to 119, further comprising: sterilizing the stent **100.**

121. The method of any one of embodiments 109 to 120, wherein the stent **100** comprises a gastrointestinal stent **100.**

122. The method of any one of embodiments 109 to 121, wherein the stent **100** comprises a biliary stent **100.**

### Methods of Using Stents

123. A method of using the stent **100** of any one of embodiments 1 to 108, said method comprising: removing the stent **100** from a packaging material (not shown).

124. The method of embodiment 123, wherein said removing step takes place in an operating room setting.

125. The method of embodiment 123 or 124, further comprising: packaging the stent **100** within a packaging material (not shown).

126. The method of any one of embodiments 123 to 125, further comprising: positioning the first end tubular member **10** within a first duct (not shown), and positioning the second tubular member **20** within a second duct (not shown), wherein the first duct and the second duct are (i) different portions of the same duct, or (ii) different ducts.

127. The method of embodiment 126, wherein said positioning steps comprise: positioning the first end tubular member **10** within a first duct comprising a right hepatic duct of an extrahepatic bile duct (EHBD), and positioning the second tubular member **20** within a second duct comprising a left hepatic duct of the EHBD so as to span a bifurcation in the EHBD.

128. The method of any one of embodiments 123 to 127, further comprising: positioning the first end tubular member **10** within a first duct (not shown), positioning the second tubular member **20** within a second duct (not shown), and positioning the third tubular member 60 within a third duct (not shown), wherein the first duct, the second duct and the third duct are (i) different portions of the same duct, or (ii) two or three different ducts.

129. The method of any one of embodiments 123 to 128, wherein said method is used to treat cholestasis.

One embodiment of the present invention relates to a stent as defined herein for use in the treatment and/or prevention of strictures in extrahepatic bile conducts and in particular for the prevention and/or treatment of choleostasis.

It should be understood that although the above-described stents **100**, and methods are described as "comprising" one or more features, components or steps, the above-described stents **100**, and methods may "comprise," "consists of," or "consist essentially of' any of the above-described features, components or steps of the stents **100**, and methods. Consequently, where the present invention, or a portion thereof, has been described with an open-ended term such as "comprising," it should be readily understood that (unless otherwise stated) the description of the present invention, or the portion thereof, should also be interpreted to describe the present invention, or a portion thereof, using the terms "consisting essentially of' or "consisting of' or variations thereof as discussed below.

As used herein, the terms "comprises," "comprising," "includes," "including," "has," "having," "contains", "containing," "characterized by" or any other variation thereof, are intended to encompass a non-exclusive inclusion, subject to any limitation explicitly indicated otherwise, of the recited components. For example, a stent **100** and/or method that "comprises" a list of elements (e.g., components or steps) is not necessarily limited to only those elements (or components or steps), but may include other elements (or components or steps) not expressly listed or inherent to the stent **100** and/or method.

As used herein, the transitional phrases "consists of' and "consisting of' exclude any element, step, or component not specified. For example, "consists of' or "consisting of' used in a claim would limit the claim to the components, materials or steps specifically recited in the claim except for impurities ordinarily associated therewith (i.e., impurities within a given component). When the phrase "consists of' or "consisting of' appears in a clause of the body of a claim, rather than immediately following the preamble, the phrase "consists of' or "consisting of' limits only the elements (or components or steps) set forth in that clause; other elements (or components) are not excluded from the claim as a whole.

As used herein, the transitional phrases "consists essentially of' and "consisting essentially of' are used to define a stent **100** and/or method that includes materials, steps, features, components, or elements, in addition to those literally disclosed, provided that these additional materials, steps, features, components, or elements do not materially affect the basic and novel characteristic(s) of the claimed invention. The term "consisting essentially of' occupies a middle ground between "comprising" and "consisting of'.

Further, it should be understood that the herein-described stents **100**, and/or methods may comprise, consist essentially of, or consist of any of the herein-described components, features, and steps, as shown in the figures with or without any feature(s) not shown in the figures. In other words, in some embodiments, the stents **100** and/or methods of the present invention do not have any additional features other than those shown in the figures, and such additional features, not shown in the figures, are specifically excluded from the stents **100** and/or methods. In other embodiments, the stents **100** and/or methods of the present invention do have one or more additional features that are not shown in the figures.

The present invention is further illustrated by the following examples, which are not to be construed in any way as imposing limitations upon the scope thereof. On the contrary, it is to be clearly understood that resort may be had to various other embodiments, modifications, and equivalents thereof which, after reading the description herein, may suggest themselves to those skilled in the art without departing from the spirit of the present invention and/or the scope of the appended claims.

### EXAMPLE 1

Stents **100** as described in embodiments 1 to 108 were prepared. The stents **100** were used to maintain bile flow through the extrahepatic bile duct, specifically where the right hepatic duct meets the left hepatic duct without impeding bile flow from either duct branch.

While the specification has been described in detail with respect to specific embodiments thereof, it will be appreciated that those skilled in the art, upon attaining an understanding of the foregoing, may readily conceive of alterations to, variations of, and equivalents to these embodiments. Accordingly, the scope of the present invention should be assessed as that of the appended claims and any equivalents thereto.

## Claims

1. A stent **100** comprising:
(I) a first end tubular member **10** comprising (i) a first end tubular member outer surface **11**, (ii) a first end tubular member inner surface **12**, (iii) a first end tubular member wall **13** extending between the first end tubular member outer surface **11** and the first end tubular member inner surface **12**, and (iv) a first end tubular member lumen **14** extending through the first end tubular member **10** along the first end tubular member inner surface **12**;
(II) a second tubular member **20** comprising (i) a second tubular member outer surface **21**, (ii) a second tubular member inner surface **22**, (iii) a second tubular member wall **23** extending between the second tubular member outer surface **21** and the second tubular member inner surface **22**, and (iv) a second tubular member lumen **24** extending through the second tubular member **20** along the second tubular member inner surface **22**; and
(III) a fluid-permeable section **30** extending between and connecting said first end tubular member **10** to said second tubular member **20**, said fluid-permeable section **30** (a) comprising one or more struts **31** with each strut **31** extending between and connecting said first end tubular member **10** to said second tubular member **20**, (b) allowing fluid flow from each of (i) said first end tubular member **10** and (ii) said second tubular member **20** to pass into and through said fluid-permeable section **30** to a location **80** outside an outer boundary **90** of said fluid-permeable section **30**, and (c) having a compliance that is more than (i) said first end tubular member **10**, and (ii) said second tubular member **20.**

2. The stent **100** of claim 1, wherein each of said first end tubular member **10** and said second tubular member **20** independently has (i) a tubular member length **L_{TM}** of less than about 5.0 centimeters (cm), (ii) a tubular member outer diameter **OD_{TM}** of less than about 1.0 cm, and (iii) a tubular member inner diameter **ID_{TM}** of less than about 0.8 cm.

3. The stent **100** of claim 1 or 2, wherein each of the one or more struts **31** comprises a strand **31'** of from about four to about 24 interconnected piece **37** having a zig-zag configuration.

4. The stent **100** of claim 1 or 2, wherein each of the one or more struts **31** comprises a strand **31'** comprising a single strand piece **37'**, and each of the single strand pieces **37'** are positioned a substantially equal distance from one another around a dissecting line **109** extending through the fluid-permeable section **30.**

5. The stent **100** of any one of claims 1 to 4, wherein said fluid-permeable section **30** comprising four struts **31.**

6. The stent **100** of any one of claims 1 to 5, wherein said fluid-permeable section **30** comprises from about 3 fluid-permeable section openings **39** to about 48 fluid-permeable section openings **39** that provide fluid flow from inside said tubular fluid-permeable section **30** to a location **80** outside of said tubular fluid-permeable section **30**, and each fluid-permeable section opening **39** has four opening sides **41.**

7. The stent **100** of any one of claims 1 to 6, wherein said first end tubular member **10** further comprises a first end tubular member flange **19** extending outward from said first end tubular member outer surface **11**, and said first end tubular member flange **19** comprises a first end tubular member flange connected end **191** and a first end tubular member flange engaging end **192**, the first end tubular member flange engaging end **192** being closer to the fluid-permeable section **30** than said first end tubular member flange connected end **191.**

8. The stent **100** of any one of claims 1 to 7, wherein said second tubular member **20** further comprises a second tubular member flange **29** extending outward from said second tubular member outer surface **21**, and said second tubular member flange **29** comprises a second tubular member flange connected end **291** and a second tubular member flange engaging end **292**, the second tubular member flange engaging end **292** being closer to the fluid-permeable section **30** that said second end tubular member flange connected end **291.**

9. The stent **100** of any one of claims 1 to 8, further comprising:
(IV) a third tubular member **60** comprising (i) a third tubular member outer surface **61**, (ii) a third tubular member inner surface **62**, (iii) a third tubular member wall **63** extending between the third tubular member outer surface **61** and the third tubular member inner surface **62**, and (iv) a third tubular member lumen **64** extending through the third tubular member **60** along the third tubular member inner surface **62**; and
(V) a second fluid-permeable section **70** extending between and connecting said second tubular member **20** to said third tubular member **60**, said second fluid-permeable section **70** (a) comprising one or more struts **71** with each strut **71** extending between and connecting said second tubular member **20** to said third tubular member **60**, (b) allowing fluid flow from each of (i) said second tubular member **20** and (ii) said third tubular member **60** to pass into and through said second fluid-permeable section **70** to a location **80'** outside an outer boundary **90'** of said second fluid-permeable section **70**, and (c) having a compliance that is more than (i) said second tubular member **20**, and (ii) said third tubular member **60.**

10. The stent **100** of claim 9, wherein each of the one or more struts **71** comprises (a) a strand **71'** of from about four to about 24 interconnected piece **37** having a zig-zag configuration, or (b) a strand **71'** comprising a single strand piece **37'**, and each of the single strand pieces **37'** are positioned a substantially equal distance from one another around a dissecting line **109** extending through the second fluid-permeable section **70.**

11. The stent **100** of claim 9 or 10, further comprising:
(VI) a fourth tubular member **120** comprising (i) a fourth tubular member outer surface **121**, (ii) a fourth tubular member inner surface **122**, (iii) a fourth tubular member wall **123** extending between the fourth tubular member outer surface **121** and the fourth tubular member inner surface **122**, and (iv) a fourth tubular member lumen **124** extending through the fourth tubular member **120** along the fourth tubular member inner surface **122**; and
(VII) a third fluid-permeable section **130** extending between and connecting said third tubular member **60** to said fourth tubular member **120**, said third fluid-permeable section **130** (a) comprising one or more struts **141** with each strut **141** extending between and connecting said third tubular member **60** to said fourth tubular member **120**, (b) allowing fluid flow from each of (i) said third tubular member **60** and (ii) said fourth tubular member **120** to pass into and through said third fluid-permeable section **70** to a location **80'** outside an outer boundary **90"** of said third fluid-permeable section **130**, and (c) having a compliance that is more than (i) said third tubular member **60**, and (ii) said fourth tubular member **120.**

12. The stent **100** of any one of claims 1 to 11, wherein said stent **100** comprises a polymeric material **50**, a metal material **50**, or a shape memory material having elastic properties such that when the fluid-permeable section **30** (and/or the first fluid-permeable section **70** and/or the third fluid-permeable section **130**) is compressed from an initial configuration via an outside pressure applied onto the fluid-permeable section **30** (and/or the first fluid-permeable section **70** and/or the third fluid-permeable section **130**), the fluid-permeable section **30** (and/or the first fluid-permeable section **70** and/or the third fluid-permeable section **130**) returns to its initial configuration once the outside pressure is removed (i.e., not applied).

13. The stent **100** of any one of claims 1 to 12, wherein said stent **100** comprises a gastrointestinal stent **100** or a biliary stent **100.**

14. A method of making the stent of any one of claims 1 to 13, said method comprising:
forming the first end tubular member,
forming the second tubular member, and
forming the fluid-permeable section, wherein said forming steps comprise: using a stent CAD model to design a specific stent that precisely matches a specific patient's anatomy.

15. A stent according to any of claims 1-14 for use in the treatment and/or prevention of strictures in extrahepatic bile conducts and in particular cholestasis.
